# EUROPEAN PATENT APPLICATION

(11) **EP 4 029 537 A1**
(43) Date of publication of application: **20.07.2022**
(21) Application number: 21152322.0
(22) Date of filing: 19.01.2021
(51) Int. Cl.: A61L 27/22, A61L 27/38, A61L 27/50

(54) **ENGINEERED HEART TISSUE PATCH, APPARATUS AND METHOD FOR PRODUCING THE SAME**

(71) Applicant: Universitätsklinikum Hamburg-Eppendorf, 20246 Hamburg (DE)
(72) Inventor: Eschenhagen, Prof. Dr. med. Thomas, 20257 Hamburg (DE); Reinsch, Dr. rer. nat. Marina, 22303 Hamburg (DE); Weinberger, Dr. med. Florian, 20257 Hamburg (DE); Mannhardt, Dr. Ingra, 20257 Hamburg (DE); Nejahsie, Dr. Yusuf, 20099 Hamburg (DE); Aksehirlioglu, Bülent, 22453 Hamburg (DE); Castro, Dr. med. Liesa, 20249 Hamburg (DE); Köse, Deniz, 22549 Hamburg (DE)
(74) Representative: Zimmermann & Partner Patentanwälte mbB

(57) **Abstract**

Described is an engineered heart tissue (EHT) patch which is suitable for use in cardiac tissue implantation, particularly into a human heart and/or onto a surface of a heart. The patch has a three-dimensional tissue structure comprising cardiomyocytes in a density of ≥ 1.5 x 10⁶ cardiomyocytes / 100 µl tissue. A method for producing the same, and an apparatus for carrying out said method is also described.

## Description

### TECHNICAL FIELD

Embodiments of the present disclosure relate to an engineered heart tissue (EHT) patch, a method of producing the same, and an apparatus for carrying out said method.

### BACKGROUND

Heart transplantation has been the most successful modality in the treatment of severe chronic heart failure. However, widespread applicability is limited by the chronic shortage of donor organs. One alternative concept is to graft heart muscle produced ex vivo by tissue engineering. Tissue engineering was first introduced as a method for rebuilding organs in the early 1990s, and the first investigations of engineered heart tissues began around the start of the new millennium. Tissue engineering generally aims at generating functional three-dimensional tissues outside of the body that can be tailored in size, shape, and function according to the respective needs before implanting them into the body. This approach may theoretically allow for complete replacement of diseased myocardium or reconstitution of missing cardiac structures in individuals with cardiac malformations.

So far, prior art methods for producing a functional heart tissue have been fraught with problems. In particular, reproducing the special organizational, mechanical, and elastic properties of native myocardium in dimensions suitable for human use represents a significant challenge from the perspective of tissue engineering scaffolds.

Thus, there exists a need to provide engineered heart tissue that displays functional and morphological properties of native myocardium and remains viable after implantation.

### SUMMARY

In the light of the above, an engineered heart tissue (EHT) patch which is suitable for use in cardiac tissue implantation as well as a method for producing the same, and an apparatus for carrying out said method is provided.

According to one embodiment, an engineered heart tissue (EHT) patch is provided which is suitable for use in cardiac tissue implantation. The patch has a three-dimensional tissue structure comprising cardiomyocytes, particularly in a density of ≥ 1.5 x 10⁶ cardiomyocytes / 100 µl tissue.

According to another embodiment, an apparatus for producing an engineered heart tissue (EHT) patch is provided. The apparatus comprises a casting vessel which is particularly suitable for holding a cell matrix suspension, and a holder which is particularly suitable for holding an EHT patch. The holder can have a three-dimensional main body comprising a plurality of posts extending from said main body. The three-dimensional main body can particularly comprise a plurality of posts extending from said main body and a through hole. The apparatus can further comprise a pin-module having a three-dimensional main body and a plurality of pins extending from said main body.

According to a further embodiment, a method for producing an engineered heart tissue (EHT) patch is provided. The method comprises the steps of providing a casting vessel; providing, in the casting vessel, a cell matrix suspension comprising fibrinogen in a concentration of ≥ 0.6 mg / 100 µl and cardiomyocytes; and culturing the cell matrix suspension for a time sufficient to form an engineered heart tissue (EHT) patch. The method can further comprise the step of removing the thus formed EHT patch from the casting vessel.

### BRIEF DESCRIPTION OF THE DRAWINGS

So that the manner in which the above recited features of the present disclosure can be understood in detail, a more particular description of the disclosure, briefly summarized above, may be had by reference to embodiments. The accompanying drawings relate to embodiments of the disclosure and are described in the following:
- FIG. 1-A: shows an apparatus for producing an engineered heart tissue patch according to embodiments described herein;
- FIG. 1-B: shows a holder of an apparatus for producing an engineered heart tissue patch according to embodiments described herein;
- FIG. 1-C: shows a casting vessel suitable for holding cell matrix suspension according to embodiments described herein;
- FIG. 1-D: shows a pin-module of an apparatus for producing an engineered heart tissue patch according to embodiments described herein;
- FIGS. 2-A and 2-B: show an apparatus for culturing an engineered heart tissue patch according to further embodiments described herein;
- FIG. 3: shows a flow chart illustrating a method for producing an engineered heart tissue patch according to embodiments described herein;
- FIG. 4-A: shows a holder with an engineered heart tissue patch according to embodiments described herein;
- FIGS. 4-B to 4-D: show an exemplary engineered heart tissue patch after detachment from a holder having a predetermined hole pattern according to embodiments described herein and its use in a porcine heart; and
- FIG. 5: shows conventional small-EHT and stripe EHT patches as well as an exemplary EHT patch having a predetermined hole pattern according to embodiments described herein.

### DETAILED DESCRIPTION

Reference will now be made in detail to the various embodiments of the disclosure, one or more examples of which are illustrated in the figures. Within the following description of the drawings, the same reference numbers refer to same components. Generally, only the differences with respect to individual embodiments are described. Each example is provided by way of explanation of the disclosure and is not meant as a limitation of the disclosure. Further, features illustrated or described as part of one embodiment can be used on or in conjunction with other embodiments to yield yet a further embodiment. It is intended that the description includes such modifications and variations.

The term "engineered heart tissue" or "EHT" as used herein refers to three-dimensional heart tissues derived by experimental manipulation of cells, in particular, pluripotent stem cells. The term "pluripotent stem cells" may be understood as cells that have the capacity to self-renew by dividing and to develop into the three primary germ cell layers of the early embryo and therefore into all cells of the adult body, except from extra-embryonic tissues such as the placenta. Examples of pluripotent stem cells are human embryonic stem cells and human induced-pluripotent stem cell (hiPSC). The term "cardiomyocytes" refers to muscle cells, in particular, myocytes, that produce heart or cardiac muscle. The terms "cell" or "cells" in the present disclosure refers to any cell that is able to produce heart or cardiac muscle or an engineered heart tissue, unless otherwise indicated. Further, the term "engineered heart tissue patch" or "EHT patch" refers to a piece of engineered heart tissue used to treat or repair injuries or damages to a heart.

Several benefits associated with the implantation of EHT patches have been observed in both small- and large-animal models of heart injury. In order to be suitable for use, particularly in humans, an EHT patch needs to be sufficiently large to cover injured or scarred areas of the heart (e.g. after a heart attack). In particular, a large EHT patch may be suitable for covering injured or scarred areas of a human heart. Further, the EHT patch has to be mechanically stable and elastic enough to be surgically manipulated and sewn on the heart, and to resist strong contraction movements of the heart. In particular, the EHT patch has to show tear resistant properties while being sewn on the heart and subsequently during the ingrowth into the recipient heart. Furthermore, the cell density of the EHT patch has to be sufficiently high so that there are enough heart muscle cells in the EHT patch to compensate for the defect of, e.g., an infarcted heart.

So far, known EHT patches could not be produced in an appropriate size to sufficiently cover an injured area of a human heart, particularly, a large injured or scarred area of a human heart. Further, in some cases, the mechanical stability of such known engineered heart tissue patches have been found to be unsuitable to allow surgically manipulation and/or sewing into a heart and/or onto a surface of a heart and also to withstand the mechanical forces of a beating heart, in particular the mechanical forces of a beating human heart. Furthermore, it has been found that mechanical stability of an engineered heart tissue having a large volume can only be achieved with excessive use of external fixings or holders to avoid disruption from the external fixings or holders during culturing, particularly those engineered heart tissues of larger dimensions, particularly in those cases where the concentration of extracellular matrix proteins originating an EHT patch is low. However, the use of such external fixings or holders may impair the cell density of an EHT patch since an excessive number of large through holes is created in the resulting EHT patch.

On the other hand, a high concentration of extracellular matrix proteins undermines the capacity of cardiomyocytes in the matrix to connect with each other, form a tissue, and develop contractile force in the resulting engineered heart tissue patch considerably. Further, an engineered heart tissue patch may break down during culture when the concentration of the extracellular matrix protein is too elevated. The present inventors have observed that the difficulty in producing large volume engineered heart tissue patches consists mainly in the optimization of a balanced combination and concentration of extracellular matrix proteins and cells in order to achieve a high mechanical stability without imparing the contractile capacity of the resulting engineered heart tissue patch. Moreover, the present inventors further identified that an appropiate apparatus and method for an optimized cell matrix suspension, e.g., including extracellular matrix proteins and cells, should first be developed accordingly.

In view of the foregoing, the present inventors found that there is still a need for improving the mechanical and elastic properties of EHT patches. Further, it is still necessary to identify the optimal combination and concentration of cells and extracellular matrix proteins, apparatus, and methods that allow providing an EHT patch suitable to be used for cardiac tissue implantation, particularly into a heart and/or onto a surface of a heart, and more particularly into a human heart and/or onto a surface of a human heart. In particular, the present inventors found that it is still necessary to identify the optimal conditions (e.g. the optimal concentration of cells and extracellular matrix proteins), apparatus, and methods for creating EHT patches having a high cell density in a large volume, particularly, a cell density of > 1.5 × 10⁶ cells / 100 µl and a volume of ≥ 15 cm³. The present inventors found that such large EHT patches may be particularly suitable for covering injured or scarred areas of a human heart.

The present disclosure provides an apparatus for producing an EHT patch comprising a casting vessel suitable for holding cell matrix suspension and a holder suitable for holding an EHT patch. The holder has a three-dimensional main body comprising a plurality of posts extending from the three-dimensional main body, particularly a plurality of posts extending from the three-dimensional main body and a through hole, and more particularly a plurality of posts extending from the three-dimensional main body and a substantially central through hole. Further, the present disclosure provides a method for producing an EHT patch which comprises the steps of providing a casting vessel, providing, particularly in the casting vessel, a cell matrix suspension comprising fibrinogen in a concentration of ≥ 0.6 mg / 100 µl and cardiomyocytes, and culturing the cell matrix suspension for a time sufficient to form an EHT patch.

The EHT patch according to embodiments described herein can show good mechanical stability and elastic properties. In particular, the EHT patch according to embodiments described herein can readily be surgically manipulated and sewn into a heart and/or onto a heart, particularly in a human heart and/or on a human heart. Further, once sewn into a heart and/or onto a heart, particularly into a human heart and/or onto a human heart, the EHT patch according to embodiments described herein can resist strong contraction movements of the heart and can compensate for heart defects based on its unique cell density. Further benefits of embodiments described herein are described in more detail in the present disclosure.

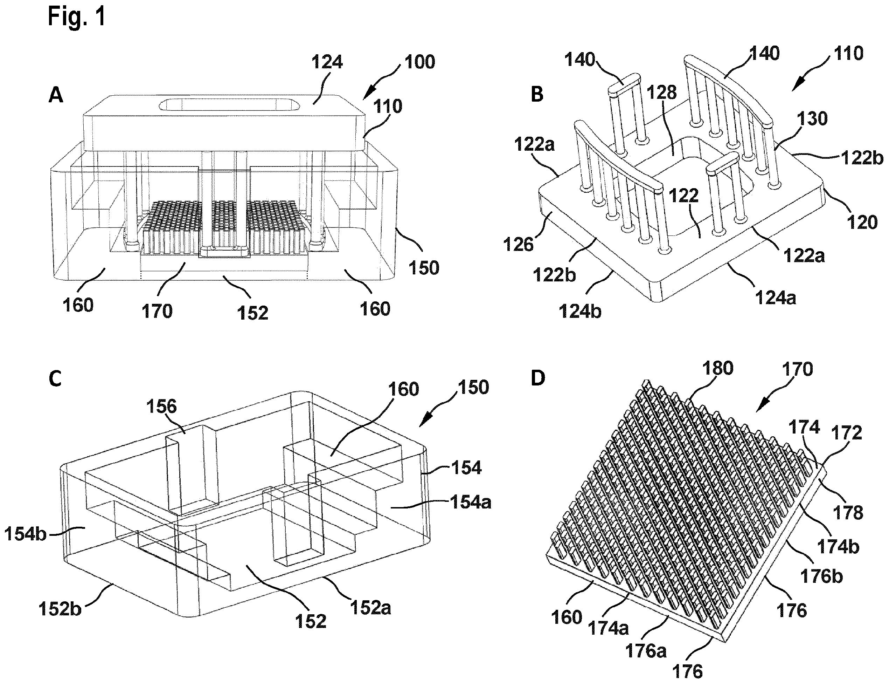

According to an embodiment of the present disclosure, an apparatus 100 for producing an engineered heart tissue patch is illustrated in Fig. 1-A. According to some embodiments, the apparatus 100 for producing an engineered heart tissue patch may comprise a holder 110 suitable for holding an EHT patch. In some embodiments, the holder 110 may comprise, or even essentially consist of, an elastic material, particularly at least one elastic material selected from the group consisting of silicone, such as polydimethylsiloxane, PVC, polyacrylate, polymethacrylate, polytetrafluoroethylene, and combinations thereof. In particular embodiments, the holder 110 may comprise, or even essentially consist of polydimethylsiloxane.

According to an embodiment of the present disclosure, the holder 110 is illustrated in more detail in Fig. 1-B. According to some embodiments, the holder 110, which is suitable for holding an EHT patch, may have a three-dimensional main body 120 comprising a plurality of posts 130 extending from the three-dimensional main body 120, particularly a plurality of posts 130 extending from the three-dimensional main body 120 and a through hole, and more particularly a plurality of posts 130 extending from the three-dimensional main body 120 and a substantially central through hole. The through hole may be useful for monitoring the development of an EHT path and for providing additional cell matrix suspension during culturing. In some embodiments, the plurality of posts 130 extending from the three-dimensional main body 120 may extend in the same direction from the three-dimensional main body 120. In some embodiments, the three-dimensional main body 120 and/or the plurality of posts 130 may comprise, or even substantially consist of, an elastic material, particularly at least one elastic material selected from the group consisting of silicone, such as polydimethylsiloxane, PVC, polyacrylate, polymethacrylate, polytetrafluoroethylene, and combinations thereof. In particular embodiments, the three-dimensional main body 120 and/or the plurality of posts 130 may comprise, or even essentially consist of polydimethylsiloxane. The elasticity of the material of the plurality of posts 130 allows for a natural contraction of a resulting EHT patch.

In some embodiments, the plurality of posts 130 may have an elongated shape. In further embodiments, the plurality of posts 130 may have at least one shape selected from the group consisting of a cylindrical shape, and a prismatic shape, such as rectangular prismatic shape, pentagonal prismatic shape or hexagonal prismatic shape, and combinations thereof. In some embodiments, the plurality of posts 130 may comprise proximal ends and distal ends. According to some embodiments, the plurality of posts 130 may have proximal ends connected to the three-dimensional main body 120. According to further embodiments, the plurality of posts 130 may have distal ends connected to each other via a bridge 140.

In some embodiments, the plurality of posts 130 may have a length or a distance between a proximal end and a distal end of 10 mm or above, particularly 20 mm or above, and more particularly 25 mm or above. According to another embodiment, the plurality of posts 130 may have a length or a distance between a proximal end and a distal end of 50 mm or below, particularly 40 mm or below, and more particularly 35 nm or below. According to yet a further embodiment, the plurality of posts 130 may have a length or a distance between a proximal end and a distal end of 10 mm or above and of 50 mm or below, particularly a length or a distance between a proximal end and a distal end of 20 mm or above and of 40 mm or below, and more particularly a length or a distance between a proximal end and a distal end of 25 mm or above and of 35 mm or below.

In some embodiments, the plurality of posts 130 may have a thickness of 1 mm or above, particularly 2 mm or above, and more particularly 2.5 mm or above. According to another embodiment, the plurality of posts 130 may have a thickness of 6 mm or below, particularly 5 mm or below, and more particularly 4 nm or below. According to yet a further embodiment, the plurality of posts 130 may have a thickness of 1 mm or above and of 6 mm or below, particularly a thickness of 2 mm or above and of 5 mm or below, and more particularly a thickness of 2.5 mm or above and of 4 mm or below. The term "thickness" in relation to the plurality of posts 130 refers to the length of a straight, cross-sectional line that is perpendicular to the length of a post of the plurality of posts 130, said line passing from one side of the post via the center to the other side.

In some embodiments, the plurality of posts 130 may have a substantially uniform thickness along their entire lengths. As used in the present disclosure, "substantially uniform thickness" is understood particularly when referring to the thickness of one post portion, e.g., the distal end of a post, in relation to the thickness of another post portion of the same post, e.g., the proximal end of said post, to allow for a thickness variation of ±20% or below, e.g. of ±10% or below, or even of ±5% or below.

In some embodiments, the plurality of posts 130 may include groups of posts. In some embodiments, distal ends of the posts of each group of posts may be connected to each other via a bridge 140. In some embodiments, each group of posts may include at least two posts, particularly at least four posts, and more particularly at least six posts. In some embodiments, the plurality of posts 130 may comprise groups of posts each having the same number of posts or a combination of different numbers of posts. The term "bridge" refers to structural means that connect at least two distal ends of at least two posts of the plurality of posts, thereby forming the groups of posts.

In some embodiments, a separation distance between the plurality of posts 130 inside each group can be 4 mm or above, particularly 6 mm or above, and more particularly 8 mm or above. In some embodiments, a separation distance between the plurality of posts 130 inside each group can be 19 mm or below, particularly 17 mm or below, and more particularly 14 mm or below. In some embodiments, a separation distance between the plurality of posts 130 inside each group can be 4 mm or above and 19 mm or below, particularly 6 mm or above and 17 mm or below, and more particularly 8 mm or above and of 14 mm or below. The term "separation distance" refers to a distance between two closest centers of cross-sectional lines perpendicular to the lengths of the corresponding posts of a plurality of posts 130, said lines passing from one side of the posts via the center of the posts to the other side of the posts.

According to some embodiments, the three-dimensional main body 120 may have a first major surface 122, a second major surface 124 opposite to the first major surface 122, and an outer side surface 126. In some embodiments, the three-dimensional main body 120 may also have an inner side surface 128. In some embodiments, the inner side surface 128 may border a through hole of the three-dimensional main body 120. In some embodiments, the outer side surface 126 may connect the first major surface 122 and the second major surface 124 with each other. In some embodiments, the inner side surface 128 may also connect the first major surface 122 and the second major surface 124 with each other. In some embodiments, the outer side surface 126 may extend substantially perpendicularly to at least one of the first major surface 122 and the second major surface 124. In some embodiments, the inner side surface 128, if present, may extend substantially perpendicularly to at least one of the first major surface 122 and the second major surface 124.

As used in the present disclosure, "substantially perpendicular" is understood particularly when referring to the orientation of the outer side surface 126 and the inner side surface 128, respectively in relation to at least one of the first major surface 122 and the second major surface 124, to allow for a deviation from the perpendicular direction or orientation of ±20° or below, e.g. of ±10° or below, or even of ±5° or below.

In some embodiments, the first major surface 122 and the second major surface 124 may have the same shape and area. In some embodiments, the first major surface 122 and the second major surface 124 may have a square shape, particularly a square shape with a through hole, more particularly a square shape with a substantially central through hole. In some embodiments, the first major surface 122 and the second major surface 124 may have a rectangular shape, particularly a rectangular shape with a central through hole, and more particularly a rectangular shape with a substantially central through hole. In some embodiments, the first major surface 122 and the second major surface 124 may have a circular or oval shape, particularly a circular or oval shape with a central through hole, and more particularly a circular or oval shape with a substantially central through hole.

According to some embodiments, the plurality of posts 130 extends substantially perpendicular from the first major surface 122 of the holder 110. As used throughout the present disclosure, "substantially perpendicular" is understood particularly when referring to the plurality of posts 130 being oriented in relation to the three-dimensional main body 120 or the first major surface 122 to allow for a deviation from the perpendicular direction or orientation of ±20° or below, e.g. of ±10° or below, or even of ±5° or below.

In some embodiments, the first major surface 122 and the second major surface 124 each may have a first pair of opposite sides 122a and 124a of 30 mm or above, particularly 50 mm or above, and more particularly 70 mm or above. In some embodiments, the first major surface 122 and the second major surface 124 each may have a first pair of opposite sides 122a and 124a of 120 mm or below, particularly 110 mm or below, and more particularly 90 mm or below. In some embodiments, the first major surface 122 and the second major surface 124 each may have a first pair of opposite sides 122a and 124a of 30 mm or above and of 120 mm or below, particularly a first pair of opposite sides 122a and 124a of 50 mm or above and of 110 mm or below, and more particularly a first pair of opposite sides 122a and 124a of 70 mm or above and of 90 mm or below. In some embodiments, the sides of the first pair of opposite sides 122a and 124a are substantially parallel to each other.

In some embodiments, the first major surface 122 and the second major surface 124 each may have a second pair of opposite sides 122b and 124b of 30 mm or above, particularly 40 mm or above, and more particularly 55 mm or above. In some embodiments, the first major surface 122 and the second major surface 124 each may have a second pair of opposite sides 122b and 124b of 115 mm or below, particularly 100 mm or below, and more particularly 85 mm or below. In some embodiments, the first major surface 122 and the second major surface 124 each may have a second pair of opposite sides 122b and 124b of 30 mm or above and of 115 mm or below, particularly a second pair of substantially parallel opposite sides 122b and 124b of 40 mm or above and of 100 mm or below, and more particularly a second pair of opposite sides 122b and 124b of 55 mm or above and of 85 mm or below. In some embodiments, the sides of the second pair of opposite sides 122b and 124b are substantially parallel to each other.

As used in the present disclosure, "substantially parallel" is understood particularly when referring to the orientation of one side of the first or second pairs of opposite sides, respectively in relation to the other side of the first or second pairs of opposite sides, respectively, to allow for a deviation from the parallel orientation of ±20° or below, e.g. of ±10° or below, or even of ±5° or below.

The terms "first pair of opposite sides" and "second pair of opposite sides" refer to outer sides 122a, 122b, 124a, and 124b of the first major surface 122 and the second major surface 124, respectively, particularly, having a rectangular shape. Further, according to some embodiments, each of the first major surface 122 and the second major surface 124 may have a through hole.

According to some embodiments, the plurality of posts 130 may be aligned, on the first major surface 122, along at least one side of the first pair of opposite sides122a, and particularly along both sides of the first pair of opposite sides 122a. According to some embodiments, the plurality of posts 130 may be aligned, on the first major surface 122, along at least one side of the second pair of opposite sides 122b of the first major surface 122, and particularly along both sides of the second pair of opposite sides 122b. According to some embodiments, the plurality of posts 130 may be aligned, on the first major surface 122, along both sides of the first pair of opposite sides 122a and further along both sides of the second pair of opposite sides 122b. According to some embodiments, the plurality of posts 130 may have proximal ends connected to the first major surface 122. In some embodiments, six posts of the plurality of posts 130 may be aligned along each side of the first pair of opposite sides 122a of the first major surface 122. In some embodiments, two posts of the plurality of posts 130 may be aligned along each side of the second pair of opposite sides 122b of the first major surface 122.

As indicated above, the three-dimensional main body 120 of the holder 110 may comprise a through hole which may be positioned substantially centrally in relation to the first and/or second major surfaces. As used in the present disclosure, "substantially centrally" is understood particularly when referring to the orientation of the through hole in relation to the first and/or second major surfaces such that the through hole at least covers the center of area of the first and/or second major surfaces.

In some embodiments, the through hole may have a shape selected from the group consisting of a polygonal shape, such as a rectangular shape (e.g., a square shape), a circular shape, and an oval shape. In some embodiments, an area of the through hole can be 10 cm² or above, particularly 12 cm² or above, and more particularly 14 cm² or above. In some embodiments, an area of the through hole can be 20 cm² or below, particularly 18 cm² or below, and more particularly 16 cm² or below. In some embodiments, an area of the through hole can be 10 cm² or above and 20 cm² or below, particularly 12 cm² or above and 18 cm² or below, and more particularly 14 cm² or above and of 16 cm² or below.

According to some embodiments, the first major surface 122 and the second major surface 124 can be substantially parallel to each other. As used in the present disclosure, "substantially parallel" is understood particularly when referring to the orientation of the first major surface 122 in relation to the second major surface 124, to allow for a deviation from the parallel orientation of ±20° or below, e.g. of ±10° or below, or even of ±5° or below.

In some embodiments, a separation distance between the first major surface 122 and the second major surface 124 can be 5 mm or above, particularly 7 mm or above, and more particularly 9 mm or above. In some embodiments, a separation distance between the first major surface 122 and the second major surface 124 can be 16 mm or below, particularly 14 mm or below, and more particularly 12 mm or below. In some embodiments, a separation distance between the first major surface 122 and the second major surface 124 can be 5 mm or above and 16 mm or below, particularly 7 mm or above and 14 mm or below, and more particularly 9 mm or above and of 12 mm or below. In some embodiments, the separation distance between the first major surface 122 and the second major surface 124 may correspond to a width of the outer side surface 126 and/or a width of the inner side surface 128, respectively.

According to some embodiments, the apparatus 100 for producing an engineered heart tissue patch may comprise a casting vessel 150 suitable for holding cell matrix suspension, as e.g. illustrated in Figs. 1-A and 1-C. In some embodiments, the casting vessel 150 may comprise at least two interior stairs 160 positioned opposite to each other. In some embodiments, the at least two opposite interior stairs may vertically extend from a base 152. In some embodiments, the at least two opposite interior stairs can be attached to the base 152 and a vertically-extending wall 154. In some embodiments, the at least two opposite interior stairs may include steps having a rise being 3 mm or above, particularly 4 mm or above, and more particularly 5 mm or above. In some embodiments, the at least two opposite interior stairs may include steps having a rise being 18 mm or below, particularly 16 mm or below, and more particularly 14 mm or below. In some embodiments, the at least two opposite interior stairs may include steps having a rise being 3 mm or above and 18 mm or below, particularly 4 mm or above and 16 mm or below, and more particularly 5 mm or above and 14 mm or below. In some embodiments, the at least two opposite interior stairs may include steps having a run being 4 mm or above, particularly 6 mm or above, and more particularly 8 mm or above. In some embodiments, the at least two opposite interior stairs may include steps having a run being 16 mm or below, particularly 14 mm or below, and more particularly 12 mm or below. In some embodiments, the at least two opposite interior stairs may include steps having a run being 4 mm or above and 16 mm or below, particularly 6 mm or above and 14 mm or below, and more particularly 8 mm or above and 12 mm or below. In some embodiments, the casting vessel 150 may comprise, or even substantially consist of, a material with anti-adhesive properties, particularly at least one material with anti-adhesive properties selected from the group consisting of agarose, polytetrafluoroethylene, and combinations thereof. In particular embodiments, the casting vessel 150 may comprise, or even essentially consist of agarose. The anti-adhesive properties of the material of the casting vessel 150 allows for an easy separation of the EHT patch from the casting vessel 150.

In some embodiments, the vertically-extending wall 154 may extend substantially perpendicular to the base 152. In some embodiments, the base 152 may comprise a horizontal base 152. In some embodiments, the at least two opposite interior stairs may horizontally-extend along at least one of the first pair of the opposite wall portions 154a and second pair of the opposite wall portions 154b of the base 152. The at least two opposite interior stairs 160 provides some space reduction in the casting vessel 150 and, therefore, the amount of a cell matrix suspension necessary for cell culturing is advantageously reduced.

As used in the present disclosure, "substantially perpendicular" is understood particularly when referring to a vertically-extending wall 154 orientation in relation to a base 152, to allow for a deviation from the perpendicular direction or orientation of ±20° or below, e.g. of ±10° or below, or even of ±5° or below. In some embodiments, the base 152 may have a shape selected from the group including a square shape or a rectangular shape. In some embodiments, the base 152 may be flat.

In embodiments with a base 152 having a rectangular shape, the base 152 may have a first pair of opposite sides 152a and a second pair of opposite sides 152b. The first pair of opposite sides 152a of the base 152 may extend 5 cm or above, particularly 7 cm or above, and more particularly 9 cm or above. In some embodiments, the first pair of opposite sides 152a of the base 152 may extend 16 cm or below, particularly 14 cm or below, and more particularly 12 cm or below. In some embodiments, first pair of opposite sides 152a of the base 152 may extend 5 cm or above and 16 cm or below, particularly 7 cm or above and 14 cm or below, and more particularly 9 cm or above and of 12 cm or below.

In some embodiments, the second pair of opposite sides 152b of the base 152 may extend 4 cm or above, particularly 4.5 cm or above, and more particularly 5 cm or above. In some embodiments, the second pair of opposite sides 152b of the base 152 may extend 10 cm or below, particularly 8 cm or below, and more particularly 7 cm or below. In some embodiments, the second pair of opposite sides 152b of the base 152 may extend 4 cm or above and 10 cm or below, particularly 4.5 cm or above and 8 cm or below, and more particularly 5 cm or above and 7 cm or below.

In some embodiments, the base 152 may have an interior area, bounded by the vertically-extending wall 154 and the at least two interior opposite stairs 160, which is 19 cm² or above, particularly 21 cm² or above, and more particularly 23 cm² or above. In some embodiments, the interior area of base 152 can be 30 cm² or below, particularly 28 cm² or below, and more particularly 26 cm² or below. In some embodiments, the interior area of base 152 can be 19 cm² or above and 30 cm² or below, particularly 21 cm² or above and 28 cm² or below, and more particularly 23 cm² or above and 26 cm² or below. The term "interior area" in relation to the base 152 refers to the area of the base 152 enclosed by the vertically-extending wall 154 and the at least two interior opposite stairs.

In some embodiments, the vertically-extending wall 154 may have a thickness of 2 mm or above, particularly 3 mm or above, and more particularly 4 mm or above. In some embodiments, the vertically-extending wall 154 may have a thickness of 10 mm or below, particularly 8 mm or below, and more particularly 6 mm or below. In some embodiments, the vertically-extending wall 154 may have a thickness of 2 mm or above and 10 mm or below, particularly 3 mm or above and 8 mm or below, and more particularly 4 mm or above and 6 mm or below.

In some embodiments, the vertically-extending wall 154 may comprise a first pair of opposite wall portions 154a and a second pair of opposite wall portions 154b. The first pair of opposite wall portions 154a may extend horizontally along the first pair of opposite sides 152a of a base 152 having a rectangular shape. The second pair of opposite wall portions 154b may extend horizontally along the second pair of opposite sides 152b of a base 152.

According to some embodiments, the casting vessel 150 may further comprise means for holding a holder. In some embodiments, the vertically-extending wall 154 may have at least two recesses 156 distal to the base 152 and suitable for holding a holder 110. According to some embodiments, the at least two recesses 156 may be positioned opposite to each other. In some embodiments, the at least two recesses 156 may at least partially extend toward the base 152. In some embodiments, the at least two recesses 156 may extend toward the base 152 20 mm or above, particularly 22 mm or above, and more particularly 24 mm or above. In some embodiments, the at least two recesses 156 may extend toward the base 152 32 mm or below, particularly 30 mm or below, and more particularly 28 mm or below. In some embodiments, the at least two recesses 156 may extend toward the base 152 20 mm or above and 32 mm or below, particularly 22 mm or above and 30 mm or below, and more particularly 24 mm or above and 28 mm or below. In some embodiments, the at least two recesses 156 may at least partially extend horizontally along at least one of the first pair of opposite wall portions 154a.

In some embodiments, the vertically-extending wall 154 may extend 2 cm or above, particularly 2.5 cm or above, and more particularly 3 cm or above from the base 152. In some embodiments, the vertically-extending wall 154 may extend 6 cm or below, particularly 5 cm or below, and more particularly 4 cm or below from the base 152. In some embodiments, the vertically-extending wall 154 may extend 2 cm or above and 6 cm or below, particularly 2.5 mm or above and 5 mm or below, and more particularly 3 mm or above and 4 mm or below from the base 152.

According to an embodiment of the present disclosure, and as e.g., illustrated in Figs. 1-A and 1-D, the apparatus for producing an engineered heart tissue patch may comprise a pin-module 170. The pin-module 170 allows for generating an engineered heart tissue patch having multiple through holes which particularly form a predetermined hole pattern. This predetermined hole pattern contributes to a homogeneous diffusion of nutrients over the entire engineered heart tissue patch and, additionally, improves the alignment of the cells and their homogeneous distribution within the engineered heart tissue patch along force lines of contraction. Further, as exemplarily illustrated in Figs. 1-A, the at least two interior opposite stairs 160 contribute to the mechanical stability of the pin-module 170 inside the casting vessel 150 since any horizontal movement of the pin-module 170 inside the casting vessel 150 is hindered. In some embodiments, the base 152 can be configured to receive and/or retain the pin-module 170, particularly to receive and/or retain the pin-module 170 in the center of the casting vessel 150.

In some embodiments, the pin-module 170 may have a three-dimensional main body 172 and a plurality of pins 180 extending substantially perpendicular from the three-dimensional main body 172. In some embodiments, the three-dimensional main body 172 of the pin-module 170 may have a first major surface 174, a second major surface 176 opposite the first major surface 174, and an outer side surface 178. In some embodiments, the outer side surface 178 may connect the first major surface 174 and the second major surface 176 with each other. In some embodiments, the outer side surface 178 may border the first major surface 174 and the second major surface 176. In some embodiments, the outer side surface 178 may extend substantially perpendicularly to at least one of the first major surface 174 and the second major surface 176.

As used in the present disclosure, "substantially perpendicular" is understood particularly when referring to the outer side surface 178 orientation in relation to at least one of the first major surface 174 and the second major surface 176, to allow for a deviation from the perpendicular direction or orientation of ±20° or below, e.g. of ±10° or below, or even of ±5° or below.

In some embodiments, the first major surface 174 and the second major surface 176 may have the same shape and area. In some embodiments, the first major surface 174 and the second major surface 176 may have a rectangular shape, which may be a square shape. In some embodiments, the first major surface 174 and the second major surface 176 may each have an area of 19 cm² or above, particularly 21 cm² or above, and more particularly 23 cm² or above. In some embodiments, the first major surface 174 and the second major surface 176 may each have an area of 31 cm² or below, particularly 29 cm² or below, and more particularly 27 cm² or below. In some embodiments, the first major surface 174 and the second major surface 176 may each have an area of 19 cm² or above and 31 cm² or below, particularly 21 cm² or above and 29 cm² or below, and more particularly 23 cm² or above and 27 cm² or below.

In some embodiments, the first major surface 174 and the second major surface 176 may have a first pair of opposite sides 174a and 176a, respectively, of 30 mm or above, particularly 40 mm or above, and more particularly 45 mm or above. In some embodiments, the first major surface 174 and the second major surface 176 may have a first pair of opposite sides 174a and 176a, respectively, of 80 mm or below, particularly 70 mm or below, and more particularly 60 mm or below. In some embodiments, the first major surface 174 and the second major surface 176 may have a first pair of opposite sides 174a and 176a, respectively, of 30 mm or above and of 80 mm or below, particularly of 40 mm or above and of 70 mm or below, and more particularly of 45 mm or above and of 60 mm or below.

In some embodiments, the first major surface 174 and the second major surface 176 may have a second pair of opposite sides 174b and 176b, respectively, of 30 mm or above, particularly 40 mm or above, and more particularly 45 mm or above. In some embodiments, the first major surface 174 and the second major surface 176 may have a second pair of opposite sides 174b and 176b, respectively, of 80 mm or below, particularly 70 mm or below, and more particularly 60 mm or below. In some embodiments, the first major surface 174 and the second major surface 176 may have a second pair of opposite sides 174b and 176b, respectively, of 30 mm or above and of 80 mm or below, particularly of 40 mm or above and of 70 mm or below, and more particularly of 45 mm or above and of 60 mm or below. The terms "first pair of opposite sides" and "second pair of opposite sides" refer to sides of the first major surface 174 and the second major surface 176, respectively, each having a rectangular shape.

In some embodiments, the plurality of pins 180 may have an elongated shape. In some embodiments, the plurality of pins 180 having an elongated shape can have a length defined by a longitudinal axis and a width defined by a lateral axis. In some embodiments, the length of the plurality of pins 180 having an elongated shape can be 1 mm or above, particularly 1.5 mm or above, and more particularly 2 mm or above. In some embodiments, the length of the plurality of pins 180 having an elongated shape can be 3.5 mm or below, particularly 3 m or below, and more particularly 2.5 mm or below. In some embodiments, the length of the plurality of pins 180 having an elongated shape can be 1 mm or above and 3.5 mm or below, particularly 1.5 mm or above and 3 mm or below, and more particularly 2 mm or above and of 2.5 mm or below In some embodiments, the width of the plurality of pins 180 having an elongated shape can be 0.3 mm or above, particularly 0.6 mm or above, and more particularly 0.9 mm or above. In some embodiments, the width of the plurality of pins 180 having an elongated shape can be 2 mm or below, particularly 1.5 m or below, and more particularly 1.2 mm or below. In some embodiments, the width of the plurality of pins 180 having an elongated shape can be 0.3 mm or above and 2 mm or below, particularly 0.6 mm or above and 1.5 mm or below, and more particularly 0.9 mm or above and of 1.2 mm or below. In further embodiments, the plurality of pins 180 may have at least one shape selected from the group consisting of a cylindrical shape, and a prismatic shape, such as rectangular prismatic shape, pentagonal prismatic shape or hexagonal prismatic shape, and combinations thereof. In some embodiments, the plurality of pins 180 may comprise proximal ends and distal ends. According to some embodiments, the plurality of pins 180 may have proximal ends connected to the three-dimensional main body 172. In some embodiments, the plurality of pins 180 of the pin-module 170 can be homogenously distributed on the three-dimensional main body 172. In some embodiments, each of pins of the plurality of pins 180 may have a cross-sectional area having a shape selected from the group consisting of a polygonal shape, such as a rectangular shape (e.g., a square shape), a circular shape, a star shape, and an oval shape. In some particular embodiments, the shape of the cross-sectional area can be oval or rectangular. In some embodiments, the plurality of pins 180 can be oriented along a direction of contraction of a resulting EHT patch. For instance, the plurality of pins 180 shown in Fig. 1-D are oriented along a direction of contraction of a resulting EHT patch.

According to some embodiments, the plurality of pins 180 may have proximal ends connected to the first major surface 174. According to some embodiments, the plurality of pins 180 of the pin-module 170 extends substantially perpendicular from the first major surface 174 of the pin-module 170. In some embodiments, the plurality of pins 180 of the pin-module 170 can be homogenously distributed on the first major surface 174. Such a homogeneous distribution particularly allows for the formation of a predetermined hole pattern in the EHT patch as described herein including the discussed benefits thereof.

In some embodiments, the plurality of pins 180 may have a length or a distance between a proximal end and a distal end of 3 mm or above, particularly 4 mm or above, and more particularly 6 mm or above. According to another embodiment, the plurality of pins 180 may have a length or a distance between a proximal end and a distal end of 11 mm or below, particularly 9 mm or below, and more particularly 8 mm or below. According to yet a further embodiment, the plurality of pins 180 may have a length or a distance between a proximal end and a distal end of 3 mm or above and of 11 mm or below, particularly a length or a distance between a proximal end and a distal end of 4 mm or above and of 9 mm or below, and more particularly a length or a distance between a proximal end and a distal end of 6 mm or above and of 8 mm or below.

In some embodiments, the plurality of pins 180 may have a thickness of 0.3 mm or above, particularly 0.5 mm or above, and more particularly 1 mm or above. According to another embodiment, the plurality of pins 180 may have a thickness of 5 mm or below, particularly 4 mm or below, and more particularly 3 nm or below. According to yet a further embodiment, the plurality of pins 180 may have a thickness of 0.3 mm or above and of 5 mm or below, particularly a thickness of 0.5 mm or above and of 4 mm or below, and more particularly a thickness of 1 mm or above and of 3 mm or below. The term "thickness" in relation to the plurality of pins 180 refers to the length of a straight, cross-sectional line that is perpendicular to the length of a pin of the plurality of pins 180, said line passing from one side of the pin via the center to the other side.

In some embodiments, a separation distance between each other of the plurality of pins 180 can be 0.3 mm or above, particularly 0.5 mm or above, and more particularly 1 mm or above. In some embodiments, a separation distance between each other of the plurality of pins 180 can be 4 mm or below, particularly 3 mm or below, and more particularly 2 mm or below. In some embodiments, a separation distance between each other of the plurality of pins 180 can be 0.3 mm or above and 4 mm or below, particularly 0.5 mm or above and 3 mm or below, and more particularly 1 mm or above and 2 mm or below.

In some embodiments, the plurality of pins 180 may include a total number of 150 or above, particularly 165 or above, and more particularly 180 or above. In some embodiments, the plurality of pins 180 may include a total number of 220 or below, particularly 210 or below, and more particularly 200 or below. In some embodiments, the plurality of pins 180 may include a total number of 150 or above and 220 or below, particularly 165 or above and 210 or below, and more particularly 180 or above and 200 or below.

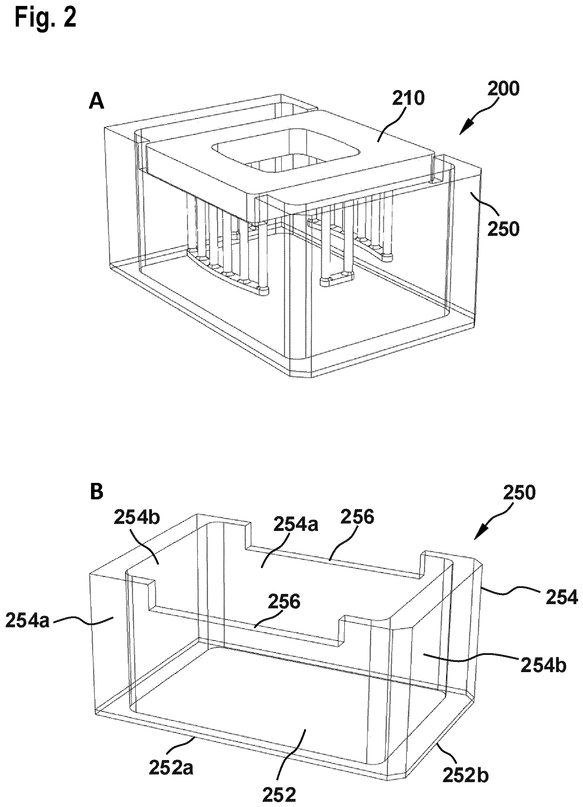

Figs. 2-A and 2-B shows an apparatus 200 for culturing an engineered heart tissue patch according to further embodiments described herein. According to some embodiments, apparatus 200 may comprise a cell culture vessel 250. According to some embodiments, the apparatus 200 may be used to culture and/or store an EHT patch. The cell culture vessel 250 may comprise a base 252 with a vertically-extending wall 254 bounding the base 252. In some embodiments, the base 252 may comprise a horizontal base 252. In some embodiments, the vertically-extending wall 254 may extend substantially perpendicularly to the base 252. In some embodiments, the vertically-extending wall 254 may border the horizontal base 252.

As used in the present disclosure, "substantially perpendicular" is understood particularly when referring to a vertically-extending wall 254 orientation in relation to a base 252, to allow for a deviation from the perpendicular direction or orientation of ±20° or below, e.g. of ±10° or below, or even of ±5° or below. In some embodiments, the base 252 may have a shape selected from the group including a square shape or a rectangular shape. In some embodiments, the base 252 may be flat.

In embodiments with a base 252 having a rectangular shape, the horizontal base 252 may have a first pair of opposite sides 252a and a second pair of opposite sides 252b. The first pair of opposite sides 252a of the base 252 may extend 5 cm or above, particularly 6 cm or above, and more particularly 8 cm or above. In some embodiments, the first pair of opposite sides 252a of the base 252 may extend 14 cm or below, particularly 12 cm or below, and more particularly 10 cm or below. In some embodiments, first pair of opposite sides 252a of the base 252 may extend 5 cm or above and 14 cm or below, particularly 6 cm or above and 12 cm or below, and more particularly 8 cm or above and 10 cm or below.

In some embodiments, the second pair of opposite sides 252b of the base 252 may extend 4 cm or above, particularly 4.5 cm or above, and more particularly 5 cm or above. In some embodiments, the second pair of opposite sides 252b of the base 252 may extend 10 cm or below, particularly 8 cm or below, and more particularly 7 cm or below. In some embodiments, the second pair of opposite sides 252b of the base 252 may extend 4 cm or above and 10 cm or below, particularly 4.5 cm or above and 8 cm or below, and more particularly 5 cm or above and 7 cm or below.

In some embodiments, the base 252 may have an interior area, bounded by the vertically-extending wall 254, which is 24 cm² or above, particularly 26 cm² or above, and more particularly 28 cm² or above. In some embodiments, the interior area of base 252 can be 36 cm² or below, particularly 34 cm² or below, and more particularly 32 cm² or below. In some embodiments, the interior area of base 252 can be 24 cm² or above and 36 cm² or below, particularly 26 cm² or above and 34 cm² or below, and more particularly 28 cm² or above and 32 cm² or below. The term "interior area" in relation to the base 252 refers to the area of the base 252 enclosed by the vertically-extending wall 254.

In some embodiments, the vertically-extending wall 254 may have a thickness of 2 mm or above, particularly 3 mm or above, and more particularly 4 mm or above. In some embodiments, the vertically-extending wall 254 may have a thickness of 10 mm or below, particularly 8 mm or below, and more particularly 6 mm or below. In some embodiments, the vertically-extending wall 254 may have a thickness of 2 mm or above and 10 mm or below, particularly 3 mm or above and 8 mm or below, and more particularly 4 mm or above and 6 mm or below.

In some embodiments, the vertically-extending wall 254 may comprise a first pair of opposite wall portions 254a and a second pair of opposite wall portions 254b. The first pair of opposite wall portions 254a may extend horizontally along the first pair of opposite sides 252a of a base 252 having a rectangular shape. The second pair of opposite wall portions 254b may extend horizontally along the second pair of opposite sides 252b of a base 252.

According to some embodiments, the cell culture vessel 250 may further comprise means for holding a holder. In some embodiments, the vertically-extending wall 254 may have at least two recesses 256 distal to the base 252 and suitable for holding a holder 210. According to some embodiments, the at least two recesses 256 may be positioned opposite to each other. In some embodiments, the at least two recesses 256 may at least partially extend toward the base 252. In some embodiments, the at least two recesses 256 may at least partially extend horizontally along at least one of the first pair of opposite wall portions 254a and second pair of opposite wall portions 254b.

In some embodiments, the vertically-extending wall 254 may extend 2 cm or above, particularly 2.5 cm or above, and more particularly 3 cm or above from the base 252. In some embodiments, the vertically-extending wall 254 may extend 6 cm or below, particularly 5 cm or below, and more particularly 4 cm or below from the base 252. In some embodiments, the vertically-extending wall 254 may extend 2 cm or above and 6 cm or below, particularly 2.5 mm or above and 5 mm or below, and more particularly 3 mm or above and 4 mm or below from the base 252. In some embodiments, the base 252 may be transparent. The transparence of the base 252 may be useful for monitoring the development of an EHT path and for analyzing contractions of the EHT path.

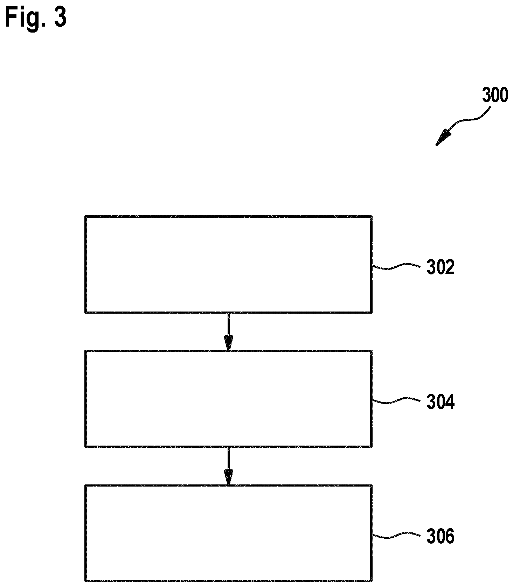

Fig. 3 shows a flow chart illustrating a method 300 for producing an engineered heart tissue patch according to embodiments described herein. According to an aspect of the present disclosure, the method 300 may include providing a casting vessel, such as the casting vessel 150 as described herein (block 302). According to embodiments described herein, the method 300 may further include providing, in the casting vessel, a cell matrix suspension comprising fibrinogen in a concentration of > 0.6 mg / 100 µl and cardiomyocytes, particularly fibrinogen in a concentration of ≥ 1.2 mg/100 µl and cardiomyocytes, and more particularly fibrinogen in a concentration of ≥ 1.5 mg/100 µl and cardiomyocytes (block 304). According to further embodiments, the method 300 may further include culturing the cell matrix suspension for a time sufficient to form an engineered heart tissue patch (block 306). The casting vessel can be any of those described above.

In some embodiments, providing, in the casting vessel, a cell matrix suspension comprising fibrinogen and cardiomyocytes may include providing a suspension comprising cardiomyocytes first and adding to the cell suspension a certain amount of fibrinogen such that the fibrinogen concentration in the resulting cell matrix suspension is ≥ 0.6 mg/100 µl, particularly ≥ 1.2 mg/100 µl, and more particularly ≥ 1.5 mg/100 µl. In other embodiments, providing, in the casting vessel, a cell matrix suspension comprising fibrinogen and cardiomyocytes may include providing a suspension comprising fibrinogen in a certain concentration first and adding to the fibrinogen suspension a cell suspension of cardiomyocytes such that the fibrinogen concentration in the resulting cell matrix suspension is ≥ 0.6 mg/100 µl, particularly ≥ 1.2 mg/100 µl, and more particularly ≥ 1.5 mg/100 µl.

In some embodiments, before culturing the cell matrix suspension for a time sufficient, a fibrinogen crosslinking agent, particularly an enzyme, and more particularly thrombin, may be added. However, it is to be understood that embodiments of the present disclosure are not limited to fibrinogen as crosslinking agent or extracellular matrix protein and any other extracellular matrix protein such as collagen, elastin, or fibronectin can be used instead or in addition to fibrinogen. According to further embodiments, culturing the cell matrix suspension for a time sufficient to form an engineered heart tissue (EHT) patch may include removing the ETH patch from the casting vessel. In some embodiments, culturing the cell matrix suspension for a time sufficient to form an engineered heart tissue (EHT) patch may include agitating the cell matrix suspension in the casting vessel. In some embodiments, culturing the cell matrix suspension for a time sufficient to form an engineered heart tissue (EHT) patch may include culturing in the casting vessel at a temperature of 30 °C to 42 °C, particularly at a temperature of 36 °C to 38°C, more particularly at a temperature of 37 °C. In some embodiments, the cardiomyocytes concentration in the cell matrix suspension, typically prior to adding a fibrinogen crosslinking agent, can be ≥ 1.5 x 10⁶ cardiomyocytes/100µl, particular ≥ 1.8 x 10⁶ cardiomyocytes/100 µl, and more particularly ≥ 2.0 x 10⁶ cardiomyocytes/100 µl.

In some embodiments, the method 300 for producing an engineered heart tissue patch may further comprise placing into the casting vessel a pin-module, such as the pin module 270 as described herein, and a holder, such as the holder 110 as described herein, before providing the cell matrix suspension in the casting vessel. In some embodiments, culturing the cell matrix suspension for a time sufficient to form an engineered heart tissue (EHT) patch may include transferring the holder, such as the holder 110 as described herein, and the engineered heart tissue patch to a cell culture vessel, such as the cell culture vessel 250 as described herein. In some embodiments, transferring the holder and the engineered heart tissue patch to a cell culture vessel may be conducted after 60 minutes of providing, in the casting vessel, a cell matrix suspension comprising fibrinogen and cardiomyocytes, particularly after 50 minutes of providing, in the casting vessel, a cell matrix suspension comprising fibrinogen and cardiomyocytes, more particularly after 40 minutes of providing, in the casting vessel, a cell matrix suspension comprising fibrinogen and cardiomyocytes. In some embodiments, culturing the cell matrix suspension for a time sufficient to form an engineered heart tissue (EHT) patch may include providing, in the cell culture vessel, a cell culture medium. In some embodiments, culturing the cell matrix suspension for a time sufficient to form an engineered heart tissue (EHT) patch may further include culturing in a cell culture vessel for a time period of up to 40 days, particularly up to 50 days, more particularly up to 60 days. In some embodiments, culturing the cell matrix suspension for a time sufficient to form an engineered heart tissue (EHT) patch may further include agitating the cell culture medium in the cell culture vessel. In some embodiments, culturing the cell matrix suspension for a time sufficient to form an engineered heart tissue (EHT) patch may also include culturing in the cell culture vessel at a temperature of 30 °C to 42 °C, particularly at a temperature of 36 °C to 38°C, more particularly at a temperature of 37 °C. In some embodiments, culturing the cell matrix suspension for a time sufficient to form an engineered heart tissue (EHT) patch may further include culturing in the cell culture vessel at a CO₂ level of 2% CO₂ to 12% CO₂, particularly at a CO₂ level of 4% O₂ to 10% O₂, more particularly at an CO₂ level of 7% O₂. In some embodiments, culturing the cell matrix suspension for a time sufficient to form an engineered heart tissue (EHT) patch may further include culturing in the cell culture vessel at an oxygen level of 20% O₂ to 50% O₂, particularly at an oxygen level of 25% O₂ to 45% O₂, more particularly at an oxygen level of 40% O₂. In some embodiments, culturing the cell matrix suspension for a time sufficient to form an engineered heart tissue (EHT) patch may also include removing the engineered heart tissue patch from the casting vessel.

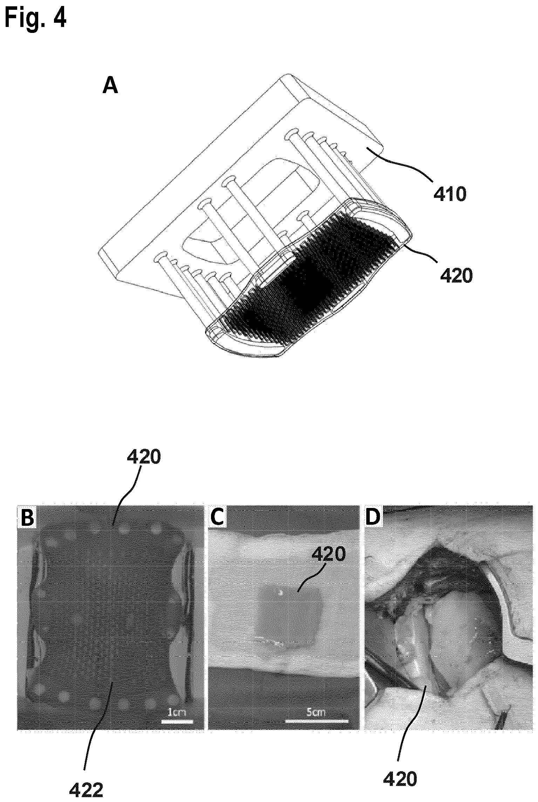

Fig. 4-A illustrates a holder 410 with an engineered heart tissue patch 420 according to embodiments described herein. According to an aspect of the present disclosure, the engineered heart tissue patch 420 can be suitable for use in cardiac tissue implantation, particularly into a heart and/or onto a surface of a heart, and more particularly into a human heart and/or onto a surface of a human heart. In some embodiments, the engineered heart tissue patch 420 may have a three-dimensional tissue structure comprising cardiomyocytes in a density of ≥ 1.5 x 10⁶ cardiomyocytes/100 µl tissue, particularly ≥ 1.8 x 10⁶ cardiomyocytes /100 µl tissue, and more particularly ≥ 2.0 x 10⁶ cardiomyocytes/100 µl tissue. In some embodiments, the engineered heart tissue patch 420, particularly having a three-dimensional tissue structure, may comprise a predetermined hole pattern in the center thereof. In some embodiments, the predetermined hole pattern may comprise elongated holes having a cross-sectional area of at least 1 mm², particularly a cross-sectional area of at least 1.5 mm², more particularly a cross-sectional area of at least 2 mm². In some embodiments, the predetermined hole pattern may include elongated holes oriented along a direction of contraction of an EHT patch.

According to some embodiments, the elongated holes of the predetermined hole pattern can have a length defined by a longitudinal axis and a width defined by a lateral axis. As will be appreciated, the elongated holes can have a primary aspect ratio of length:width such that the length is greater than the width. In accordance with an embodiment, the primary aspect ratio of length: width can be at least 1.1:1, at least 1.2:1, at least 1.5:1, at least 1.8:1, at least 2:1, at least 3:1, at least 4:1, at least 5:1, at least 6:1, or even at least 10:1. In another embodiment, the elongated holes can have a primary aspect ratio of length:width of not greater than 20:1, not greater than 15:1, not greater than 10:1, not greater than 6:1, not greater than 5:1, not greater than 4:1, not greater than 3:1, or even not greater than 2:1. It will be appreciated that the primary aspect ratio of the elongated holes can be with a range including any of the minimum and maximum ratios noted above. It will be appreciated that not all holes are elongated holes, and some non-shaped holes can be substantially equiaxed, such that any combination of the length and width are substantially the same.

According to some embodiments, the engineered heart tissue patch 420 may have a volume of ≥ 5 cm³, particularly a volume of ≥ 15 cm³, and more particularly a volume of ≥ 20 cm³. In some embodiments, the engineered heart tissue patch 420 may have at least one shape selected from the group consisting of a polyhedral shape, a cylindrical shape, a cuboid shape, a prismatic shape, a pyramidal shape, and a parallelepipedal shape. In some embodiments, the engineered heart tissue patch 420 may have a cuboid shape with a dimension of length (1) x width (w) x height (h) of ≥ 3.5 cm x 3.5 cm x 0.4 cm, particularly, ≥ 6 cm x 5 cm x 0.4 cm, and more particularly ≥ 10 cm x 7 cm x 0.5 cm.

According to some embodiments, the engineered heart tissue patch 420 may comprise a total amount of ≥ 1.5 x 10⁸ cardiomyocytes, particularly a total amount of ≥ 3 x 10⁸ cardiomyocytes, and more particularly a total amount of ≥ 4 x 10⁸ cardiomyocytes. In some embodiments, the engineered heart tissue patch 420 may have a weight of ≥ 4.5 g, particularly a weight of ≥ 10 g, and more particularly a weight of ≥ 35 g.

Figs. 4-B to 4-D illustrate an engineered heart tissue patch 420 after detachment from a holder 410 according to embodiments described herein. The engineered heart tissue patch 420 was obtained after 40 days of culturing in a cell culture vessel. A predetermined hole pattern 422 can be observed in the engineered heart tissue patch 420. Further, the predetermined hole pattern 422 includes elongated holes oriented along a direction of contraction of the EHT patch. As exemplarily illustrated in Figs. 4-B and 4-C, the structure of the engineered heart tissue patch 420 is kept after detachment from the holder 410. In particular, the engineered heart tissue patch 420 illustrated in Fig. 4-C has the following dimensions: 6 cm x 5 cm x 0.4 cm. The engineered heart tissue patch 420 illustrated in Figs. 4-B and 4-C can be easily handled to be implanted to a pig heart. As exemplarily illustrated in Fig. 4-D, the engineered heart tissue patch 420 can be sewed on a pig heart without showing any tear. Further, the engineered heart tissue patch 420 can cover the anterior wall of a pig heart.

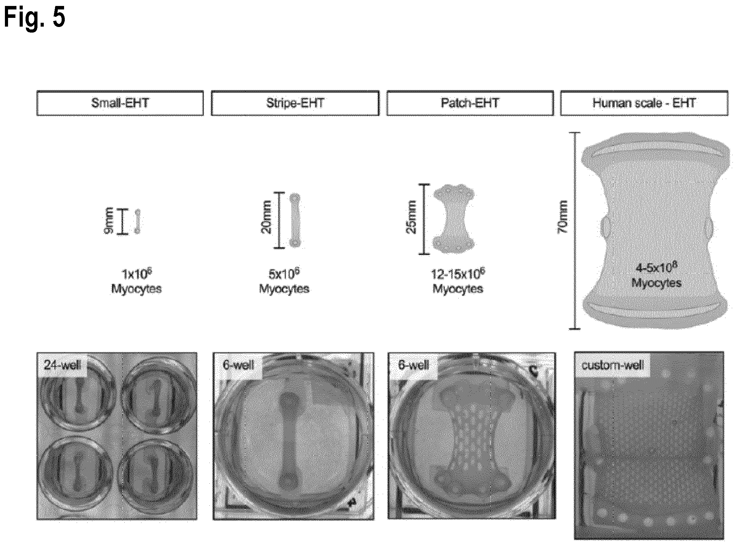

FIG. 5 illustrates two conventional EHT patches (i.e. a so-called "Small-EHT Patch" and a "Stripe-EHT Patch") in comparison to an exemplary EHT patch according to embodiments described herein. The human scale EHT patch according to embodiments described herein is, due to its unique architecture, particularly suitable for being implanted into a human heart and/or onto a surface of a human heart. The unique architecture of the human scale EHT patch is particularly characterized by its size in combination with an outstanding total amount of cells (a high cell density) and a predetermined hole pattern.

The present disclosure has several advantages including the production of a large, artificially produced heart muscle (i.e. an engineered heart tissue (EHT) patch) which is suitable for use in cardiac tissue implantation, particularly into a human heart and/or onto a surface of a human heart. The EHT patch described herein shows good mechanical stability and elastic properties. It can resist strong contraction movements of the heart and shows a unique cell density which is sufficiently high to compensate for the defects of a damaged heart. For instance, the EHT patch according to embodiments herein can typically be produced with a size of ca. 5 cm x 7 cm and with a total amount of at least 450 million cardiomyocytes, which is about 15% of the total number of cardiomyocytes of a human being. Despite such high cardiomyocyte densities, no significant death of cardiomyocytes in the middle of the tissues was observed. The EHT patch further shows a homogenous cell distribution throughout its three-dimensional tissue structure. This allows for excellent contractibility. Due to its large size, the EHT patch according to embodiments herein can be easily removed from the apparatus for producing the engineered heart tissue patch with the use of tweezers, and can be easily sewn in a heart and/or on the surface of a heart, particularly in a human heart and/or on the surface of a human heart, using standard suture materials. Due to this, the reproducibility of a cardiac tissue implantation of the EHT patch into a heart and/or onto the surface of a heart, particularly into a human heart and/or onto the surface of a human heart, is significantly improved. Handling and suturing of EHT patches described herein on the epicardial surface of human hearts is technically easy, demonstrating thereby proving the surgical feasibility of this approach. The EHT patches described herein are suitable to withstand the mechanical forces during implantation, and also resist the mechanical forces of the heart once implanted.

While the foregoing is directed to embodiments of the disclosure, other and further embodiments of the disclosure may be devised without departing from the basic scope thereof, and the scope thereof is determined by the claims that follow.

### Example

An EHT patch was obtained by using an agarose casting vessel, such as the casting vessel 150 described above including a first pair of opposite sides 152a extending 7 cm and a second pair of opposite sides 152b extending 5 cm, a holder including polydimethylsiloxane and two groups of six posts (length between a proximal end and a distal end of the posts 30 mm, thickness 3 mm) on opposite sides of the holder and two groups of posts of two posts on each of the remaining sides, and a pin-module including pins (length: 2 mm, width: 1 mm, and height: 7 mm of the pins), such as the pin-module 170 described above. 430 x 106 cardiomyocytes were resuspended in 25 ml of the reconstitution mix (containing 5 mg/ml fibrinogen), briefly mixed with thrombin (100 U/ml) and pipetted into the agarose casting vessel. After ∼ 30 min at 37 °C the resulting EHT patch attached to the holder was transferred to a cell culture vessel, such as the cell culture vessel 250 described above. The EHT patch was further cultured in a cell culture medium including DMEM (Gibco 41965-039) supplemented with 1% Penicillin/Streptomycin (Gibco 15140); 10% Human Serum (Biowest S4190); 33 µg/ml Aprotinin (Sigma A1153); 2 mM Glutamine (Gibco 25030); 1% Non-essential amino acids (Gibco 11140); 100 ng/ml IGF-1 (Peprotech 100-11); 10 ng/ml FGF-2 (Peprotech 100-18B); 5 ng/ml VEGF (R&D Systems 293-VE); 5 ng/ml TGF-β (Peprotech 100-21C). Culturing conditions: 37 °C, 7% CO2, and 40% O₂. The EHT was fed daily with 90-125 ml of the previously mentioned cell culture medium. The EHT was cultured under dynamic conditions on a rocking platform with 30° tilt at 0.4 Hz for 40 days.

## Claims

1. An engineered heart tissue (EHT) patch suitable for use in cardiac tissue implantation, the patch having a three-dimensional tissue structure comprising cardiomyocytes in a density of > 1.5 x 10⁶ cardiomyocytes /100 µl tissue.

2. The EHT patch according to claim 1, wherein the volume of the patch is ≥ 5 cm³.

3. The EHT patch according to any of the preceding claims having at least one shape selected from the group consisting of a polyhedral shape, a cylindrical shape, a cuboid shape, a prismatic shape, a pyramidal shape, and a parallelepipedal shape.

4. The EHT patch according to claim 3, wherein the EHT patch has a cuboid shape with a dimension of length (1) x width (w) x height (h) of ≥ 3.5 cm x 3.5 cm x 0.4 cm.

5. The EHT patch according to any of the preceding claims comprising a total amount of ≥ 450 x 10⁶ cardiomyocytes.

6. The EHT patch according to any of the preceding claims having a weight of ≥ 4.5 g.

7. The EHT patch according to any of the preceding claims, wherein the three-dimensional tissue structure comprises a predetermined hole pattern in the center thereof.

8. The EHT patch according to any of the preceding claims, wherein the predetermined hole pattern comprises elongated holes having a cross-sectional area of at least 1 mm².

9. An apparatus for producing an engineered heart tissue (EHT) patch, comprising:
- a casting vessel suitable for holding cell matrix suspension; and
- a holder suitable for holding an EHT patch, the holder having a three-dimensional main body comprising a plurality of posts extending from the main body, and particularly comprising a plurality of posts extending from the main body and a through hole.

10. The apparatus according to claim 9, wherein the three-dimensional main body of the holder has a first major surface, a second major surface opposite the first major surface, an outer side surface, particularly a first major surface, a second major surface opposite the first major surface, an outer side surface, and an inner side surface bordering the through hole, the outer side surface connecting the first and second major surfaces with each other, wherein the plurality of posts extends substantially perpendicular from the first major surface of the holder.

11. The apparatus according to claim 10, wherein the plurality of posts includes groups of posts, wherein distal ends of the posts of each group are connected to each other via a bridge.

12. The apparatus according to any one of claims 9 to 11, wherein the holder comprises an elastic material.

13. A method for producing an engineered heart tissue (EHT) patch, comprising:
- providing a casting vessel;
- providing, in the casting vessel, a cell matrix suspension comprising fibrinogen in a concentration of > 0.6 mg/100µl and cardiomyocytes;
- culturing the cell matrix suspension for a time sufficient to form an engineered heart tissue (EHT) patch.

14. The method according to claim 13, wherein the cardiomyocyte concentration in the cell matrix suspension is ≥ 1.5 x 10⁶ cardiomyocytes/100 µl.

15. The method according to any one of claims 13 or 14, further comprising placing into the casting vessel a pin-module before providing the cell matrix suspension in the casting vessel.
